# EUROPEAN PATENT APPLICATION

(11) **EP 1 785 139 A1**
(43) Date of publication of application: **16.05.2007**
(21) Application number: 05776100.9
(22) Date of filing: 31.08.2005
(51) Int. Cl.: A61K 31/56, A61K 31/136, A61K 31/337, A61K 31/4745, A61K 31/475, A61K 31/553, A61K 45/00, A61P 35/00, A61P 43/00

(54) **ANTAGONIST AGAINST TOLERANCE TO ANTICANCER DRUGS**

(30) Priority: 31.08.2004 JP 2004251839
(71) Applicant: JAPANESE FOUNDATION FOR CANCER RESEARCH, Tokyo 135-8550 (JP); KABUSHIKI KAISHA YAKULT HONSHA, Minato-ku, Tokyo 105-8660 (JP); Sugimoto, Yoshikazu, Kashiwa-shi, Chiba, 2770061 (JP)
(72) Inventor: TSUKAHARA, Satomi, 1510073 (JP); IMAI, Yasuo, 1130022 (JP); SUGIMOTO, Yoshikazu, Kashiwa-shi, Chiba, 2770061 (JP)
(74) Representative: Blodig, Wolfgang
(86) International application number: PCT/JP2005/015873
(87) International publication number: WO 2006/025431

(57) **Abstract**

An ABC transporter protein expression inhibitor comprising, as the active ingredient(s), 0.001 to 100 nM of one or more members selected from among steroid hormones, compounds having a female hormone function, analogous compounds thereof and antagonistic inhibitors therefor; an anticancer composition containing this ABC transporter protein expression inhibitor and an anticancer drug; and cells useful in the development of an anticancer drug. The present invention provides a drug which inhibits the expression of an ABC transporter to thereby overcome resistance to anticancer drugs; cancer cells useful in screening such drugs; and an anticancer drug efficacious even against such a cancer as having acquired resistance to anticancer drugs.

SEQUENCE LISTING

<110> Sugimoto, Yoshikazu
Japanese Foundation for Cancer Research
Kabushiki Kaisha Yakult Honsha

<120> Reagents reversing anticancer drug resistance

<130> YK0033

<150> JP 2004-251839
<151> 2004-08-31

<160> 7

<170> PatentIn version 3.1

<210> 1
<211> 23
<212> DNA
<213> Artificial

<220>
<223> Designed primer for human BCRP

<400> 1
cctgagatcc tgagcctttg gtt 23

<210> 2
<211> 25
<212> DNA
<213> Artificial

<220>
<223> Designed primer for human BCRP

<400> 2
gatggcaagg gaacagaaaa caaca 25

<210> 3
<211> 1968
<212> DNA
<213> Homosapiens

<220>
<221> CDS
<222> (1)..(1968)

<223>

<400> 3
atg tct tcc agt aat gtc gaa gtt ttt atc cca gtg tca caa gga aac 48
Met Ser Ser Ser Asn Val Glu Val Phe Ile Pro Val Ser Gln Gly Asn
1               5                   10                  15

acc aat ggc ttc ccc gcg aca gct tcc aat gac ctg aag gca ttt act 96
Thr Asn Gly Phe Pro Ala Thr Ala Ser Asn Asp Leu Lys Ala Phe Thr
            20                  25                  30

gaa gga gct gtg tta agt ttt cat aac atc tgc tat cga gta aaa ctg 144
Glu Gly Ala Val Leu Ser Phe His Asn Ile Cys Tyr Arg Val Lys Leu
        35                  40                  45

aag agt ggc ttt cta cct tgt cga aaa cca gtt gag aaa gaa ata tta 192
Lys Ser Gly Phe Leu Pro Cys Arg Lys Pro Val Glu Lys Glu Ile Leu
    50                  55                  60

tcg aat atc aat ggg atc atg aaa cct ggt ctc aac gcc atc ctg gga 240
Ser Asn Ile Asn Gly Ile Met Lys Pro Gly Leu Asn Ala Ile Leu Gly
65                  70                  75                  80

ccc aca ggt gga ggc aaa tct tcg tta tta gat gtc tta gct gca agg 288
Pro Thr Gly Gly Gly Lys Ser Ser Leu Leu Asp Val Leu Ala Ala Arg
                85                  90                  95

aaa gat cca agt gga tta tct gga gat gtt ctg ata aat gga gca ccg 336
Lys Asp Pro Ser Gly Leu Ser Gly Asp Val Leu Ile Asn Gly Ala Pro
            100                 105                 110

cga cct gcc aat ttc aaa tgt aat tca ggt tac gtg gta caa gat gat 384
Arg Pro Ala Asn Phe Lys Cys Asn Ser Gly Tyr Val Val Gln Asp Asp
        115                 120                 125

gtt gtg atg ggc act ctg acg gtg aga gaa aac tta cag ttc tca gca 432
Val Val Met Gly Thr Leu Thr Val Arg Glu Asn Leu Gln Phe Ser Ala
    130                 135                 140

gct ctt cgg ctt gca aca act atg acg aat cat gaa aaa aac gaa cgg 480
Ala Leu Arg Leu Ala Thr Thr Met Thr Asn His Glu Lys Asn Glu Arg
145                 150                 155                 160

att aac agg gtc att caa gag tta ggt ctg gat aaa gtg gca gac tcc 528
Ile Asn Arg Val Ile Gln Glu Leu Gly Leu Asp Lys Val Ala Asp Ser
                165                 170                 175

aag gtt gga act cag ttt atc cgt ggt gtg tct gga gga gaa aga aaa 576
Lys Val Gly Thr Gln Phe Ile Arg Gly Val Ser Gly Gly Glu Arg Lys

            180                 185                 190

agg act agt ata gga atg gag ctt atc act gat cct tcc atc ttg ttc 624
Arg Thr Ser Ile Gly Met Glu Leu Ile Thr Asp Pro Ser Ile Leu Phe
        195                 200                 205

ttg gat gag cct aca act ggc tta gac tca agc aca gca aat gct gtc 672
Leu Asp Glu Pro Thr Thr Gly Leu Asp Ser Ser Thr Ala Asn Ala Val
    210                 215                 220

ctt ttg ctc ctg aaa agg atg tct aag cag gga cga aca atc atc ttc 720
Leu Leu Leu Leu Lys Arg Met Ser Lys Gln Gly Arg Thr Ile Ile Phe
225                 230                 235                 240

tcc att cat cag cct cga tat tcc atc ttc aag ttg ttt gat agc ctc 768
Ser Ile His Gln Pro Arg Tyr Ser Ile Phe Lys Leu Phe Asp Ser Leu
                245                 250                 255

acc tta ttg gcc tca gga aga ctt atg ttc cac ggg cct gct cag gag 816
Thr Leu Leu Ala Ser Gly Arg Leu Met Phe His Gly Pro Ala Gln Glu
            260                 265                 270

gcc ttg gga tac ttt gaa tca gct ggt tat cac tgt gag gcc tat aat 864
Ala Leu Gly Tyr Phe Glu Ser Ala Gly Tyr His Cys Glu Ala Tyr Asn
        275                 280                 285

aac cct gca gac ttc ttc ttg gac atc att aat gga gat tcc act gct 912
Asn Pro Ala Asp Phe Phe Leu Asp Ile Ile Asn Gly Asp Ser Thr Ala
    290                 295                 300

gtg gca tta aac aga gaa gaa gac ttt aaa gcc aca gag atc ata gag 960
Val Ala Leu Asn Arg Glu Glu Asp Phe Lys Ala Thr Glu Ile Ile Glu
305                 310                 315                 320

cct tcc aag cag gat aag cca ctc ata gaa aaa tta gcg gag att tat 1008
Pro Ser Lys Gln Asp Lys Pro Leu Ile Glu Lys Leu Ala Glu Ile Tyr
                325                 330                 335

gtc aac tcc tcc ttc tac aaa gag aca aaa gct gaa tta cat caa ctt 1056
Val Asn Ser Ser Phe Tyr Lys Glu Thr Lys Ala Glu Leu His Gln Leu
            340                 345                 350

tcc ggg ggt gag aag aag aag aag atc aca gtc ttc aag gag atc agc 1104
Ser Gly Gly Glu Lys Lys Lys Lys Ile Thr Val Phe Lys Glu Ile Ser
        355                 360                 365

tac acc acc tcc ttc tgt cat caa ctc aga tgg gtt tcc aag cgt tca 1152
Tyr Thr Thr Ser Phe Cys His Gln Leu Arg Trp Val Ser Lys Arg Ser
    370                 375                 380

ttc aaa aac ttg ctg ggt aat ccc cag gcc tct ata gct cag atc att 1200
Phe Lys Asn Leu Leu Gly Asn Pro Gln Ala Ser Ile Ala Gln Ile Ile
385                 390                 395                 400

gtc aca gtc gta ctg gga ctg gtt ata ggt gcc att tac ttt ggg cta 1248
Val Thr Val Val Leu Gly Leu Val Ile Gly Ala Ile Tyr Phe Gly Leu
                405                 410                 415

aaa aat gat tct act gga atc cag aac aga gct ggg gtt ctc ttc ttc 1296
Lys Asn Asp Ser Thr Gly Ile Gln Asn Arg Ala Gly Val Leu Phe Phe
            420                 425                 430

ctg acg acc aac cag tgt ttc agc agt gtt tca gcc gtg gaa ctc ttt 1344
Leu Thr Thr Asn Gln Cys Phe Ser Ser Val Ser Ala Val Glu Leu Phe
        435                 440                 445

gtg gta gag aag aag ctc ttc ata cat gaa tac atc agc gga tac tac 1392
Val Val Glu Lys Lys Leu Phe Ile His Glu Tyr Ile Ser Gly Tyr Tyr
    450                 455                 460

aga gtg tca tct tat ttc ctt gga aaa ctg tta tct gat tta tta ccc 1440
Arg Val Ser Ser Tyr Phe Leu Gly Lys Leu Leu Ser Asp Leu Leu Pro
465                 470                 475                 480

atg agg atg tta cca agt att ata ttt acc tgt ata gtg tac ttc atg 1488
Met Arg Met Leu Pro Ser Ile Ile Phe Thr Cys Ile Val Tyr Phe Met
                485                 490                 495

tta gga ttg aag cca aag gca gat gcc ttc ttc gtt atg atg ttt acc 1536
Leu Gly Leu Lys Pro Lys Ala Asp Ala Phe Phe Val Met Met Phe Thr
            500                 505                 510

ctt atg atg gtg gct tat tca gcc agt tcc atg gca ctg gcc ata gca 1584
Leu Met Met Val Ala Tyr Ser Ala Ser Ser Met Ala Leu Ala Ile Ala
        515                 520                 525

gca ggt cag agt gtg gtt tct gta gca aca ctt ctc atg acc atc tgt 1632
Ala Gly Gln Ser Val Val Ser Val Ala Thr Leu Leu Met Thr Ile Cys
    530                 535                 540

ttt gtg ttt atg atg att ttt tca ggt ctg ttg gtc aat ctc aca acc 1680
Phe Val Phe Met Met Ile Phe Ser Gly Leu Leu Val Asn Leu Thr Thr
545                 550                 555                 560

att gca tct tgg ctg tca tgg ctt cag tac ttc agc att cca cga tat 1728
Ile Ala Ser Trp Leu Ser Trp Leu Gln Tyr Phe Ser Ile Pro Arg Tyr
                565                 570                 575

gga ttt acg gct ttg cag cat aat gaa ttt ttg gga caa aac ttc tgc     1776
Gly Phe Thr Ala Leu Gln His Asn Glu Phe Leu Gly Gln Asn Phe Cys
            580                 585                 590

cca gga ctc aat gca aca gga aac aat cct tgt aac tat gca aca tgt     1824
Pro Gly Leu Asn Ala Thr Gly Asn Asn Pro Cys Asn Tyr Ala Thr Cys
        595                 600                 605

act ggc gaa gaa tat ttg gta aag cag ggc atc gat ctc tca ccc tgg     1872
Thr Gly Glu Glu Tyr Leu Val Lys Gln Gly Ile Asp Leu Ser Pro Trp
    610                 615                 620

ggc ttg tgg aag aat cac gtg gcc ttg gct tgt atg att gtt att ttc     1920
Gly Leu Trp Lys Asn His Val Ala Leu Ala Cys Met Ile Val Ile Phe
625                 630                 635                 640

ctc aca att gcc tac ctg aaa ttg tta ttt ctt aaa aaa tat tct taa     1968
Leu Thr Ile Ala Tyr Leu Lys Leu Leu Phe Leu Lys Lys Tyr Ser
                645                 650                 655


<210>  4
<211>  655
<212>  PRT
<213>  Homosapiens

<400>  4

Met Ser Ser Ser Asn Val Glu Val Phe Ile Pro Val Ser Gln Gly Asn
1               5                   10                  15


Thr Asn Gly Phe Pro Ala Thr Ala Ser Asn Asp Leu Lys Ala Phe Thr
            20                  25                  30


Glu Gly Ala Val Leu Ser Phe His Asn Ile Cys Tyr Arg Val Lys Leu
        35                  40                  45


Lys Ser Gly Phe Leu Pro Cys Arg Lys Pro Val Glu Lys Glu Ile Leu
    50                  55                  60


Ser Asn Ile Asn Gly Ile Met Lys Pro Gly Leu Asn Ala Ile Leu Gly
65                  70                  75                  80


Pro Thr Gly Gly Gly Lys Ser Ser Leu Leu Asp Val Leu Ala Ala Arg

85                  90                  95

Lys Asp Pro Ser Gly Leu Ser Gly Asp Val Leu Ile Asn Gly Ala Pro
            100                 105                 110

Arg Pro Ala Asn Phe Lys Cys Asn Ser Gly Tyr Val Val Gln Asp Asp
        115                 120                 125

Val Val Met Gly Thr Leu Thr Val Arg Glu Asn Leu Gln Phe Ser Ala
    130                 135                 140

Ala Leu Arg Leu Ala Thr Thr Met Thr Asn His Glu Lys Asn Glu Arg
145                 150                 155                 160

Ile Asn Arg Val Ile Gln Glu Leu Gly Leu Asp Lys Val Ala Asp Ser
                165                 170                 175

Lys Val Gly Thr Gln Phe Ile Arg Gly Val Ser Gly Gly Glu Arg Lys
            180                 185                 190

Arg Thr Ser Ile Gly Met Glu Leu Ile Thr Asp Pro Ser Ile Leu Phe
        195                 200                 205

Leu Asp Glu Pro Thr Thr Gly Leu Asp Ser Ser Thr Ala Asn Ala Val
    210                 215                 220

Leu Leu Leu Leu Lys Arg Met Ser Lys Gln Gly Arg Thr Ile Ile Phe
225                 230                 235                 240

Ser Ile His Gln Pro Arg Tyr Ser Ile Phe Lys Leu Phe Asp Ser Leu
                245                 250                 255

Thr Leu Leu Ala Ser Gly Arg Leu Met Phe His Gly Pro Ala Gln Glu
            260                 265                 270

Ala Leu Gly Tyr Phe Glu Ser Ala Gly Tyr His Cys Glu Ala Tyr Asn
        275                 280                 285

Asn Pro Ala Asp Phe Phe Leu Asp Ile Ile Asn Gly Asp Ser Thr Ala
    290                 295                 300

Val Ala Leu Asn Arg Glu Glu Asp Phe Lys Ala Thr Glu Ile Ile Glu
305                 310                 315                 320

Pro Ser Lys Gln Asp Lys Pro Leu Ile Glu Lys Leu Ala Glu Ile Tyr
                325                 330                 335

Val Asn Ser Ser Phe Tyr Lys Glu Thr Lys Ala Glu Leu His Gln Leu
            340                 345                 350

Ser Gly Gly Glu Lys Lys Lys Lys Ile Thr Val Phe Lys Glu Ile Ser
        355                 360                 365

Tyr Thr Thr Ser Phe Cys His Gln Leu Arg Trp Val Ser Lys Arg Ser
    370                 375                 380

Phe Lys Asn Leu Leu Gly Asn Pro Gln Ala Ser Ile Ala Gln Ile Ile
385                 390                 395                 400

Val Thr Val Val Leu Gly Leu Val Ile Gly Ala Ile Tyr Phe Gly Leu
                405                 410                 415

Lys Asn Asp Ser Thr Gly Ile Gln Asn Arg Ala Gly Val Leu Phe Phe
            420                 425                 430

Leu Thr Thr Asn Gln Cys Phe Ser Ser Val Ser Ala Val Glu Leu Phe
        435                 440                 445

Val Val Glu Lys Lys Leu Phe Ile His Glu Tyr Ile Ser Gly Tyr Tyr
    450                 455                 460

Arg Val Ser Ser Tyr Phe Leu Gly Lys Leu Leu Ser Asp Leu Leu Pro
465                 470                 475                 480

Met Arg Met Leu Pro Ser Ile Ile Phe Thr Cys Ile Val Tyr Phe Met
                485                 490                 495

Leu Gly Leu Lys Pro Lys Ala Asp Ala Phe Phe Val Met Met Phe Thr
            500                 505                 510

Leu Met Met Val Ala Tyr Ser Ala Ser Ser Met Ala Leu Ala Ile Ala
        515                 520                 525

Ala Gly Gln Ser Val Val Ser Val Ala Thr Leu Leu Met Thr Ile Cys
    530                 535                 540

Phe Val Phe Met Met Ile Phe Ser Gly Leu Leu Val Asn Leu Thr Thr
545                 550                 555                 560

Ile Ala Ser Trp Leu Ser Trp Leu Gln Tyr Phe Ser Ile Pro Arg Tyr
                565                 570                 575

Gly Phe Thr Ala Leu Gln His Asn Glu Phe Leu Gly Gln Asn Phe Cys
            580                 585                 590

Pro Gly Leu Asn Ala Thr Gly Asn Asn Pro Cys Asn Tyr Ala Thr Cys
        595                 600                 605

Thr Gly Glu Glu Tyr Leu Val Lys Gln Gly Ile Asp Leu Ser Pro Trp
    610                 615                 620

Gly Leu Trp Lys Asn His Val Ala Leu Ala Cys Met Ile Val Ile Phe
625                 630                 635                 640

Leu Thr Ile Ala Tyr Leu Lys Leu Leu Phe Leu Lys Lys Tyr Ser
                645                 650                 655

<210> 5
<211> 77
<212> DNA
<213> Artificial

<220>
<223>  Designed primer for human BCRP

<400>  5
ccccgcggca tggaacaaaa actcatctca gaagaggatc tgtcttccag taatgtcgaa      60

gtttttatcc cagtgtc                                                    77


<210>  6
<211>  34
<212>  DNA
<213>  Artificial

<220>
<223>  Designed primer for human BCRP

<400>  6
cgcctcgtgg atggcaaggg aacagaaaac aaca                                 34


<210>  7
<211>  77
<212>  DNA
<213>  Artificial

<220>
<223>  Designed primer for human BCRP

<400>  7
ccccgcggca tgtacccata cgacgtccca gactacgcta tgtcttccag taatgtcgaa      60

gtttttatcc cagtgtc                                                    77

## Description

### Technical Field

The present invention relates to anticancer drugs which are effective against cancer that has acquired anticancer drug resistance and to cells which are useful for developing such anticancer drugs.

### Background Art

Anticancer drugs such as camptothecins (e.g., irinotecan hydrochloride) and mitoxantrone exhibit surprisingly excellent effect against malignant tumors and thus have been widely employed in clinical settings. However, researchers have pointed out that a prolonged and continuous use of those drugs sometimes result in a reduction in efficacy. Recent research on the mechanism with which cancer cells acquire resistance to the anticancer drugs has revealed that BCRP, which is an ABC transporter, participates in the acquisition of anticancer drug resistance (Non-Patent Document 1). Specifically, according to the findings of the research, after a prolonged continuous use of an anticancer drug, BCRP comes to be expressed in cancer cells, and the BCRP discharges the anticancer drug out of the cells to thereby reduce the amount of anticancer drug accumulated within the cells. In this connection, p-glycoprotein encoded by MDR1 gene is also known as an ABC transporter which participates in the acquisition of anticancer drug resistance (Non-Patent Document 2). P-glycoprotein has two ATP-binding cassettes and exhibits substrate specificity different from that of BCRP.
Non-Patent Document 1: Proc. Natl. Acad. Sci. USA, 95(26), 15665-15670 (1998)
Non-Patent Document 2: Methods in Enzymology, 292: 248-594 (1998)

### Disclosure of the Invention

### Problems to be Solved by the Invention

Until today, researchers have failed to identify a low-molecular-weight compound that can prevent expression of an ABC transporter in cancer cell lines that have come to acquire anticancer drug resistance as a result of expression, or elevated expression, of the ABC transporter, and, no useful experimental system has been established. Therefore, researchers could not develop means for overcoming anticancer drug resistance, on the basis of suppression of expression of an ABC transporter.
Accordingly, the present invention provides a drug which overcomes anticancer drug resistance by preventing expression of an ABC transporter; cancer cells useful for screening candidate drugs to identify a drug which overcomes, through prevention of expression of an ABC transporter, anticancer drug resistance; and an anticancer drug which is efficacious against a cancer that has acquired anticancer drug resistance.

### Means for Solving the Problems

The present inventors have carried out screening of a variety of substances with an aim to identify a compound capable of preventing expression of BCRP through use of cancer cells which intrinsically express ABC transporters at high level, in particular BCRP at high level, and have found that very low levels of a steroid hormone, a compound having a female hormone function, an analogous compound thereof, and an antagonistic inhibitor therefor effectively lower expression of BCRP in MCF-7 cells having female hormone receptors.
The present inventors have also studied transfer of BCRP gene by using breast cancer cells bearing female hormone receptors, such as MCF-7 and T-47D, and have successfully established breast cancer cells which contain exogenous BCRP gene and which have acquired anticancer drug resistance. The inventors have further studied transfer of p-glycoprotein gene by using cells such as MCF-7 and T-47D, and have successfully established breast cancer cells which contain exogenous p-glycoprotein gene and which have acquired anticancer drug resistance.
Moreover, the present inventors have discovered that expression of BCRP or p-glycoprotein in breast cancer cells is reduced by a very low level of a steroid hormone, a compound having a female hormone function, an analogous compound thereof, or an antagonistic inhibitor therefor, to thereby effectively overcome the anticancer drug resistance. The present invention has accomplished on the basis of this finding.

Accordingly, the present invention provides an ABC transporter protein expression inhibitor comprising, as active ingredient(s) thereof, 0.001 to 100 nM of one or more members selected from among steroid hormones, compounds having a female hormone function, analogous compounds thereof and antagonistic inhibitors therefor.
The present invention also provides an anticancer composition containing such an ABC transporter protein expression inhibitor and an anticancer drug.
The present invention also provides breast cancer cells MCF-7 which exhibit resistance against 7-ethyl-10-hydroxycamptothecin (SN-38) and express BCRP at high level; breast cancer cells T-47D which exhibit resistance against 7-ethyl-10-hydroxycamptothecin (SN-38) and express BCRP at high level; breast cancer cells MCF-7 which exhibit resistance against vincristine and express p-glycoprotein at high level; and breast cancer cells T-47D which exhibit resistance against vincristine and express p-glycoprotein at high level.
The present invention further provides a method for screening ABC transporter protein expression inhibitors, which comprises using, as an indicator, expression level of BCRP or p-glycoprotein in the above-described breast cancer cells exhibiting resistance against 7-ethyl-10-hydroxycamptothecin (SN-38) or in breast cancer cells exhibiting vincristine resistance.

### Effects of the Invention

The present invention can recover the effect of anticancer drugs which are prevented from exhibiting sufficient drug efficacy because of an ABC transporter (in particular, BCRP) or p-glycoprotein being expressed. Thus, dosage of anticancer drugs can be easily controlled, to thereby realize cancer chemotherapy with minimized adverse side effects.
The invention also enables retrieval of compounds which effectively suppress expression of BCRP or p-glycoprotein, and provides a drug development system useful for elucidating the action mechanism of the retrieved compounds.

### Brief Description of the Drawings

[Fig. 1] Fig. 1 shows suppressive effect of estrone, estradiol, and diethylstilbestrol on expression of endogenous BCRP.
[Fig. 2] Fig. 2 shows suppressive effect of estradiol on expression of exogenous BCRP.
[Fig. 3] Fig. 3 shows the results of a cell growth inhibition test performed by using SN-38 on MCF-7 cells or MCF-7/MycBCRP cells.
[Fig. 4] Fig. 4 shows the results of a cell growth inhibition test performed by using SN-38 or vincristine on
MCF-7 cells or MCF-7/MycBCRP cells in the presence of estradiol.
[Fig. 5] Fig. 5 shows inhibiting effect of estradiol on expression of p-glycoprotein.

### Best Mode for Carrying Out the Invention

The present invention will be described focusing on a typical ABC transporter, BCRP.
A steroid hormone, a compound having a female hormone function, or a similar compound was added to respective cells of MCF-7 (breast cancer), A549 (lung adenocarcinoma), and JEG-3(placental choriocarcinoma), which intrinsically express BCRP at high level. As a result, as shown in Example 1, significant reduction in expression level of BCRP was observed only in MCF-7 cells expressing female hormone receptors.

Next will be described cancer cells harboring an exogenous BCRP gene.
The BCRP gene which may be transferred to cancer cells has already been registered (DDBJ accession number AB056867). It is also described in the literature (see, for example, Doyle, L. A., Yang, W., Abruzzo, L. V., Krogmann, T., Gao, Y., Rishi, A. K. and Ross, D. D. "A multidrug resistance transporter from human MCF-7 breast cancer cells" Proc. Natl. Acad. Sci. U.S.A. 95(26), 15665-15670 (1998)). No particular limitation is imposed on the BCRP gene so long as expression of BCRP is attained. For example, the following may be employed: a retrovirus-vector-inserted plasmid; i.e., pHaBCRP or pHa-BCRP-IRES-DHFR. More specifically, an especially preferred plasmid is constructed by inserting a Myc-epitope-tagged BCRP into a retrovirus vector.

Examples of preferred cancer cells include MCF-7 and T-47D cell, for the reasons that they express female hormone receptors, that they are easily cultured, and that they show sensitivity to anticancer drugs which are transported by BCRP.

An exogenous BCRP gene can be easily transferred to cancer cells according to a routine procedure, using a BCRP-gene-inserted retrovirus or a similar material.
The resultant exogenous-BCRP-gene-harboring cancer cells have acquired anticancer drug resistance, as proven by expression of BCRP and reduced intracellular uptake of anticancer drug. Therefore, the cells are useful for screening ABC transporter protein expression inhibitors. In particular, breast cancer cells that express BCRP produced through gene transfer by using a retrovirus are very useful in studies to overcome anticancer drug resistance caused by BCRP, because, as compared with parent cells, they do not affect other anticancer drug resistance genes such as MDR1 and MRP, and they are convenient in terms of handling. Such cells may be directly screened in vitro. Alternatively, they may be first transplanted to an animal such as mice, followed by in vivo screening.
Specific examples of cells which may be used for screening include breast cancer cells MCF-7 which exhibit resistance against 7-ethyl-10-hydroxycamptothecin (SN-38) and express BCRP at high level; breast cancer cells T-47D which exhibit resistance against 7-ethyl-10-hydroxycamptothecin (SN-38) and express BCRP at high level; breast cancer cells MCF-7 which exhibit resistance against vincristine and express p-glycoprotein at high level; and breast cancer cells T-47D which exhibit resistance against vincristine and express p-glycoprotein at high level.
The screening method of the present invention may be carried out as follows: breast cancer cells which exhibit resistance against 7-ethyl-10-hydroxycamptothecin (SN-38) and express BCRP at high level or breast cancer cells which exhibit resistance against vincristine and express p-glycoprotein at high level are cultured in the presence or absence with other culture conditions being unchanged of a test substance; subsequently, select, as an ABC transporter protein expression inhibitor, a test substance which brings a reduction in expression level of BCRP or p-glycoprotein.
Examples of the breast cancer cells include breast cancer cells MCF-7 and breast cancer cells T-47D. The concentration of the test substance employed for culturing is preferably 0.0001 to 100 nM, more preferably, 0.01 to 10 nM. Cultivation is carried out for, for example, 2 to 5 days. The expression level of BCRP or p-glycoprotein can be determined through, for example, Western blotting.

The above-produced cells were employed. Specifically, a steroid hormone, a compound having a female hormone function, or a similar compound was added to MCF-7 cells or T-47D cells harboring an exogenous BCRP gene. As a result, as shown in Example 2 provided hereinbelow, expression level of BCRP was significantly reduced, revealing that sensitivity to an anticancer drug; i.e., cancer cell growth inhibitory effect provided by an anticancer drug, can be recovered. Accordingly, the mentioned compounds are useful as ABC transporter protein expression inhibitors in cancer cells.

As used herein, preferred substances from among the steroid hormones, female hormones, their analogues, and antagonistic inhibitors therefor are female hormones and their analogues. Specific examples include follicle hormones such as estrone, estradiol, estradiol benzoate, estradiol dipropionate, estradiol valerate, ethinylestradiol, estriol, estriol acetate benzoate, estriol tripropionate, conjugated estrogens, mestranol, diethylstilbestrol, diethylstilbestrol dipropionate, fosfestrol, estramustine sodium phosphate, and their analogues; corpus luteum hormones such as progesterone, pregnenolone, pregnanediol, dydrogesterone, hydroxyprogesterone caproate, hydroxyprogesterone acetate, chlormadinone acetate, allylesterenol, and gestonorone caproate, and their analogues; nortestosterones such as norethisterone and allylsterol, and their analogues; and flavonoids such as genistein and naringenin, and their analogues.

The concentration of the above-listed female hormones and their analogues in ABC transporter protein expression inhibitors of the present invention is preferably 0.001 to 100 nM, more preferably 0.01 to 10 nM.

No particular limitation is imposed on anticancer drugs with which the ABC transporter protein expression inhibitor of the present invention is useful, so long as they are anticancer drugs which exhibit resistance induced by BCRP or p-glycoprotein. Examples of useful anticancer drugs include camptothecins such as irinotecan hydrochloride, topotecan, and topotecin; anthraquinones such as mitoxantrone; staurosporines such as 7-hydroxystaurosporine; anthracyclines such as doxorubicin hydrochloride, Daunomycin, epirubicin hydrochloride, and adriamycin; vinka alkaloids such as vincristine; taxanes such as paclitaxel and docetaxel; and etoposide, mitomycin, gefinitib, and imanitib.

No particular limitation is imposed on the cancer targeted by the ABC transporter protein expression inhibitor of the present invention, so long as the aforementioned anticancer drugs are used for treatment. However, the cancer cells express hormone receptors, in particular female hormone receptor, are preferred.

When (A) an ABC transporter protein expression inhibitor of the present invention is used in combination with (B) an anticancer drug which exhibits acquired cancer cell resistance, therapeutic effect against the cancer that has acquired drug resistance can be recovered, so that a composition containing these ingredients (A) and (B) is useful as a novel anticancer drug.

The ABC transporter protein expression inhibitor of the present invention or the novel anticancer drug of the present invention may be administered in such a way that conventional agents, each conventionally containing the above ingredients, may be administered in combination. Alternatively, by incorporating the above two ingredients, a new drug product may be produced. Exemplary product forms include oral administration form, injection form (including intramuscular, subcutaneous, and intravenous), suppositories, and external-use form (patches, paints, etc.).

Dose of the ABC transporter protein expression inhibitor of the present invention varies depending on the manner of administration, pathological conditions, etc. A daily dose of 0.1 to 10 mg is preferred. The dose of an anticancer drug (B) which develops drug resistance in cancer cells may be an ordinary efficacy-providing dose; for example, 1 mg to 1 g, in particular 2 to 300 mg.

### Examples

The present invention will next be described in detail by way of Examples, which should not be construed as limiting the invention thereto.

### Example 1 (Suppression of expression of endogenous BCRP)

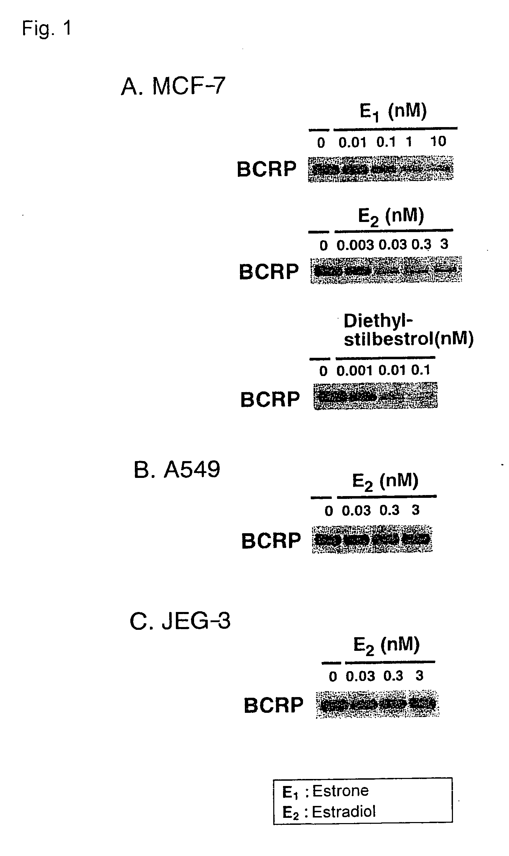

Western blotting was performed to investigate the effect of a steroid hormone and a female hormone on MCF-7, A549, and JEG-3 cells, which intrinsically express BCRP at high level, in terms of the expression level of BCRP. Estrone or estradiol was added to a phenol red-free DMEM medium containing fetal bovine serum (7%) which had been treated with activated carbon to remove steroids, and incubation was performed for 4 days. Afterwards, expression level of BCRP was determined through the Western blotting technique using an anti-BCRP antibody. In each lane, 30 µg of protein was electrophoresed.
In the presence of estrone or estradiol, expression level of endogenous BCRP in MCF-7 cells decreased to 10 to 20% the level as measured for control. However, in other cells, no such changes were observed (Fig. 1).

### Example 2

### (1) BCRP gene

In the present invention, human BCRP cDNA, which had been isolated from human placenta mRNA through PCR, was employed. In PCR, the materials employed were human placenta Marathon-ready cDNA (Clontech Co.) (as a template); 5'-side primer 1S of human BCRP cDNA (CCT GAG ATC CTG AGC CTT TGG TT) (SEQ ID No: 1) and 3'-side primer 5AS of human BCRP cDNA (GAT GGC AAG GGA ACA GAA AAC AAC A) (SEQ ID No: 2) (as two oligonucleotides serving as primers); and an Advantage cDNA PCR kit (Clontech Co.). The PCR conditions were as follows: 1 × 94°C (1 min) → 35 × {94°C (30 sec) + 68°C (3 min)} → 1 × {94°C (30 sec) + 68°C (15 min)}. As a result, an amplified cDNA of about 2,150 bp was obtained. The thus-obtained cDNA was subcloned into a PCR2.1 plasmid, and the nucleotide sequence of the cDNA was determined by means of ABI PRISM377 DNA sequencer (Applied Biosystems Co.). Sequencing of mutually independent 4 clones was performed. With any portions considered to be PCR-induced mutations having been disregarded, the nucleotide sequence of the coding region of the present gene was determined (SEQ ID No: 3). An amino acid sequence deduced therefrom is shown by SEQ ID No: 4. In the present invention, this sequence is referred to as the sequence of a wild-type BCRP. The sequence of BCRP according to the present invention is registered as DDBJ accession number AB056867 and described in JP-A-2003-63989.

### (2) Preparation of BCRP-expressing plasmid

Next, PCR was performed again in order to modify the end of the sequence so as to enable insertion of a Myc-epitope-tagged BCRP cDNA. When the PCR for addition of a Myc epitope tag was carried out, the following materials were employed: human BCRP cDNA obtained from the above PCR (as a template); 5'-side primer 5Myc-204S containing Myc epitope tag (CCC CGC GGC ATG GAA CAA AAA CTC ATC TCA GAA GAG GAT CTG TCT TCC AGT AAT GTC GAA GTT TTT ATC CCA GTG TC) (SEQ ID No: 5) and 3'-side primer 8AS (CGC CTC GTG GAT GGC AAG GGA ACA GAA AAC AAC A) (SEQ ID No: 6) (as two oligonucleotides serving as primers); and an Advantage cDNA PCR kit (Clontech Co.). The PCR conditions were as follows: 1 × 94°C (1 min) → 20 × {94°C (30 sec) + 68°C (3 min)} → 1 × {94°C (30 sec) + 68°C (15 min)}. As a result, an amplified cDNA of about 2,200 bp was obtained. The amplified cDNA was subjected to subcloning to thereby determine the nucleotide sequence and confirm that no PCR-induced mutation was present. When the PCR for addition of an HA epitope tag was carried out, the following materials were employed: human BCRP cDNA obtained from the above PCR (as a template); 5'-side primer 5HA-204S containing an HA epitope tag (CCC CGC GGC ATG TAC CCA TAC GAC GTC CCA GAC TAC GCT ATG TCT TCC AGT AAT GTC GAA GTT TTT ATC CCA GTG TC) (SEQ ID No: 7) and 3'-side primer 8AS (CGC CTC GTG GAT GGC AAG GGA ACA GAA AAC AAC A) (SEQ ID No: 6) (as two oligonucleotides serving as primers); and an Advantage cDNA PCR kit (Clontech Co.). The PCR conditions were as follows: 1 × 94°C (1 min) → 20 × {94°C (30 sec) + 68°C (3 min)} → 1 × {94°C (30 sec) + 68°C (15 min)}. As a result, an amplified cDNA of about 2,200 bp was obtained. The amplified cDNA was subjected to subcloning to thereby determine the nucleotide sequence and confirm that no PCR-induced mutation was present.
Both ends of each cDNA were digested with two restriction enzymes SstII and XhoI and were subsequently subjected to ligation with a pHa plasmid vector digested with SstII and XhoI by use of a T4 DNA ligase. The ligation reaction mixture was added to E. coli DH5α, to thereby yield clones pHaMycBCRP and pHaHABCRP, which have BCRP cDNA inserted between the SstII site and XhoI site of the pHa plasmid vector.

### (3) Preparation of BCRP retrovirus

Firstly, calcium phosphate transfection was performed to transfer pHaMycBCRP and pHaHABCRP to PA317 cells belonging to a mouse amphotropic retrovirus packaging cell line. Cells which had undergone gene transfer were subjected to selection with 1-ng/mL mitoxantrone, whereby gene-transferred cells were obtained. The supernatant of the cell culture was collected and filtered with a 0.45-µm filter, to thereby obtain a retrovirus liquid.

### (4) Preparation of MCF-7/MycBCRP cell

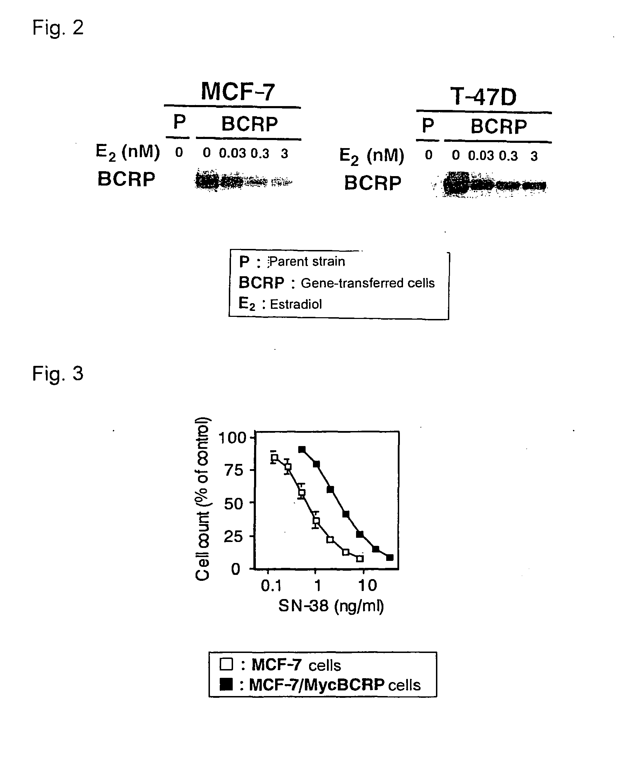

A MycBCRP retrovirus liquid was added to a culture broth of human breast cancer MCF-7 cells, whereby gene transfer was performed. Retrovirus-added cells were selected using 20-ng/mL SN-38 (7-ethyl-10-hydroxycamptothecin: an active form of irinotecan hydrochloride), to thereby produce gene-transferred cells. The cells were named MCF-7/MycBCRP. MCF-7 cells and MCF-7/MycBCRP cells were cultured in DMEM medium supplemented with 7% fetal bovine serum. Western blotting using anti-Myc antibody confirmed that BCRP protein was expressed in MCF-7/MycBCRP cells (Fig. 2). In each lane, 20µg of protein was electrophoresed. MCF-7 cells, which are human breast cancer cells, constitute a suitable parent strain of BCRP-gene transferred cells for the reasons that they intrinsically express female hormone receptors, that they can be easily cultured, and that they exhibit sensitivity to anticancer drugs transported by BCRP, such as mitoxantrone and irinotecan hydrochloride. Also, MCF-7 cells and MCF-7/MycBCRP cells can be transplanted to immunodeficient mice to thereby perform animal experiments of BCRP inhibitors and like substances.

### (5) Preparation of T-47D/MycBCRP cell

A MycBCRP retrovirus liquid was added to a culture broth of human breast cancer T-47D cells, whereby gene transfer was performed. Retrovirus-added cells were selected using 10-ng/mL SN-38, to thereby produce gene-transferred cells. The cells were named T-47D/MycBCRP. T-47D cells and T-47D/MycBCRP cells were cultured in DMEM medium supplemented with 7% fetal bovine serum. Western blotting using anti-BCRP antibody confirmed that BCRP protein was expressed in T-47D/MycBCRP cells (Fig. 2). T-47D cells, which are human breast cancer cells, constitute a suitable parent strain of BCRP-gene transferred cells for the reasons that they intrinsically express no BCRP, that they express female hormone receptors, that they can be easily cultured, and that they exhibit sensitivity to anticancer drugs transported by BCRP, such as mitoxantrone and irinotecan hydrochloride.

### Example 3 (Suppression of expression of exogenous BCRP)

Western blotting was performed to determine the expression level of BCRP in MCF-7/MycBCRP cells and T-47D/MycBCRP cells. Estradiol was added to a phenol red-free DMEM medium containing fetal bovine serum (7%) which had been treated with activated carbon to remove steroids, and incubation was performed for 4 days. Afterwards, expression level of BCRP was determined through the Western blotting technique using an anti-Myc antibody (Fig. 2).
In the presence of estradiol, expression levels of exogenous BCRP in MCF-7/MycBCRP cells and T-47D/MycBCRP cells decreased to 10 to 20% the level as measured for control (Fig. 2).

### Example 4 (Cell growth inhibition test)

A cell growth inhibition test was performed to investigate the sensitivity of MCF-7 cells and MCF-7/MycBCRP cells to SN-38. Respective cells were seeded on 12-well plates (Iwaki) in amounts of 30,000 cells/1 mL/well. Subsequently, the drug, diluted with a medium to different concentrations, was added thereto (1 mL per well). The plates were placed in a 5% CO₂ incubator and cultivation was performed at 37°C for 5 days. Four days after, a cell solution in each well was added to a beaker containing a CELLPACK diluent (9.5 mL, Toa Medical Electronics Co.). The number of cells was counted by means of a Sysmex CDA-500 automatic cell counter (Toa Medical Electronics Co.). In Fig. 3, the cell count is shown by "% of control," which was obtained by dividing "the cell count as measured when the drug diluted to have different concentrations was added" by "the cell count as measured when no such drug was added." MCF-7/MycBCRP cells exhibited a resistance of about 3 to 4 times against SN-38 (Fig. 3).

### Example 5 (Estradiol overcomes BCRP-originating resistance)

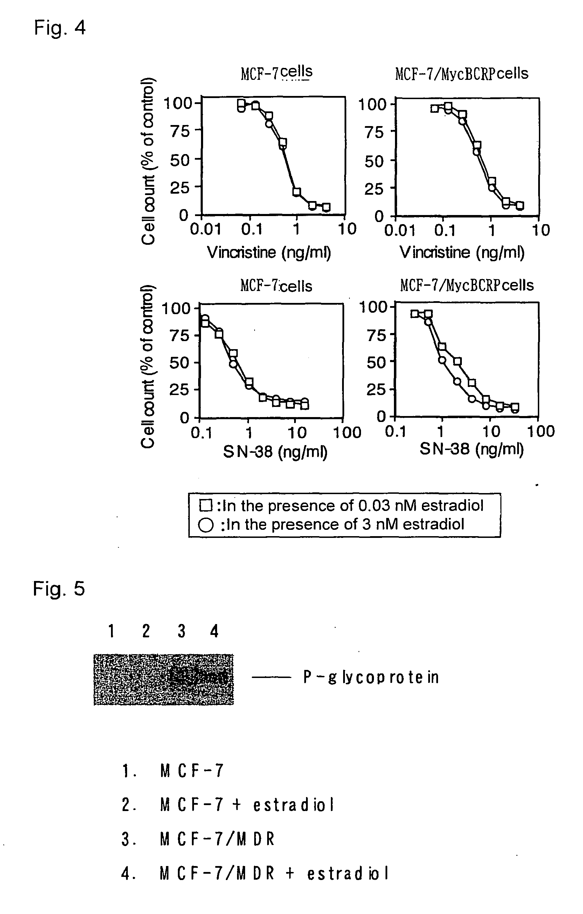

A cell growth inhibition test was performed to investigate whether estradiol induces any change in sensitivity of MCF-7 cells and MCF-7/MycBCRP cells to SN-38. Respective cells were seeded on 12-well plates (Iwaki) in amounts of 30,000 cells/1 mL/well. Subsequently, the drug, diluted with a medium to different concentrations, was added thereto (1 mL per well). The final concentration of estradiol was adjusted to 0.03 nM or 3 nM. The plates were placed in a 5% CO₂ incubator and cultivation was performed at 37°C for 4 days. Four days after, a cell solution in each well was added to a beaker containing a CELLPACK diluent (9.5 mL, Toa Medical Electronics Co.). The number of cells was counted by means of a Sysmex CDA-500 automatic cell counter (Toa Medical Electronics Co.). In Fig. 4, the cell count is shown by "% of control," which was obtained by dividing "the cell count as measured when the drug diluted to have different concentrations was added" by "the cell count as measured when no such drug was added."
Whereas the sensitivity of MCF-7 cells to SN-38 was almost the same level as that to vincristine at both of the estradiol concentrations 0.03 nM and 3 nM, MCF-7/MycBCRP cells exhibited about twice an increase in sensitivity to SN-38 at an estradiol concentration of 3 nM as compared with the sensitivity exhibited at an estradiol concentration of 0.03 nM (Fig. 4). Table 1 shows changes in sensitivity to SN-38 or vincristine, caused by addition of estradiol. The changes are shown by the concentration that inhibits cell growth by 50%.

**[Table 1]**

| Sensitivity of MCF-7 cells and MCF-7/MycBCRP cells to SN-38 or vincristine in the presence of E₂ (estradiol) | | | |
|---|---|---|---|
| Drug | E₂ (nM) | IC₅₀ (ng/mL) | |
| | | MCF-7 | MCF-7/MycBCRP |
| SN-38 | 0.03 | 0.64 ± 0.06 | 2.13 ± 0.19* |
| | 3 | 0.50 ± 0.02 | 1.09 ± 0.09* |
| Vincristine | 0.03 | 0.64 ± 0.01 | 0.68 ± 0.03 |
| | 3 | 0.60 ± 0.02 | 0.60 ± 0.02 |

| | | | |
|---|---|---|---|
| IC₅₀: Concentration at which cell growth is inhibited by 50% *: p<0.01 | | | |

### Example 6 (Suppression of expression of exogenous P-glycoprotein)

### (1) MDR1 gene

P-glycoprotein is a first ABC transporter which was identified as being related to anticancer drug resistance. The full-length cDNA sequence of a p-glycoprotein gene, human MDR1 gene, has already been reported by a research group in the U.S.A.
The gene named "MDR1" has been registered at the GenBank under accession number M14758, and has also been described in, for example, Chen, C., J., et al., "Internal duplication and homology with bacterial transport proteins in the mdr1 (P-glycoprotein) gene from multidrug-resistant human cells" Cell 47: 381-389 (1986).
However, the sequence of MDR1 cDNA was determined through use of colchicine-resistance cancer cells treated with a mutagen ethylmethane sulfonate. When compared with the MDR1 gene which is most commonly found among the Japanese (so-called wild type MDR1 gene), there have been identified the following differences: C540T, G554T, A555T, and T1236C, wherein the base of the wild type MDR1 gene is shown in the left of each numeral. Of these nucleotide sequence differences, C540T is a polymorphism present in a codon coding for the 180th serine and this gene polymorphism leads to no change in amino acid. T1236C is a polymorphism present in a codon coding for the 412th glycine and this gene polymorphism also leads to no change in amino acid. G554T and A555T relate to a mutation to valine, as CCA that encodes the 185th glycine of the wild type MDR1 gene is changed to GTT. This change occurs after the cancer cells are treated with the mutagen, and there is considered to be an artifact mutation.
In the present invention, the gene called human wild type MDR1 cDNA is isolated from a human adrenal cDNA library, which is described in Kioka, N., et al. "P-glycoprotein gene (MDR1) cDNA from human adrenal: Normal P-glycoprotein carries Gly185 with an altered pattern of multidrug resistance" Biochem Biophys Res Commun 162: 224-231 (1989).

### (2) MDR1-expressing plasmid

Wild type MDR1-expressing retrovirus vector plasmid pHaMDR employed in the present invention is described in Sigimoto, Y., Aksentijevich, I., Gottesman, M. M., and Pastan, I., "Efficient expression of drug-selectable genes in retroviral vectors under control of an internal ribosome entry site" Nature Biotechnology 12: 694-698 (1994).

### (3) Preparation of MDR1 retrovirus

The retrovirus liquid of wild type MDR1-expressing retrovirus HaMDR employed in the present invention was prepared as follows: calcium phosphate transfection was performed to introduce a pHaMDR plasmid to PA317 cells, which constitute a mouse amphotropic retrovirus packaging cell line; thereafter, 35 ng/ml vincristine was employed for selecting vincristine-resistant cells; the thus-selected cells were subjected to cloning by way of limiting dilution; and a supernatant of a culture of retrovirus-producing cells 3P26 was collected. 3P26 cells is described in Suzuki, M., Sigimoto, Y., Tsukahara, S., Okochi, E., Gottesman. M. M., and Tsuruo, T., "Retroviral co-expression of two different types of drug-resistant genes for the chemoprotection of normal cells from combinaton chemotherapy" Clin. Cancer Res., 3: 947-954 (1997).
A culture supernatant of 3P26 cells was collected and filtered through a 0.45-µm filter, whereby a retrovirus liquid was obtained.

### (4) Preparation of MCF-7/MDR1 cells

An HaMDR retrovirus liquid was added to a culture of human breast cancer cells MCF-7 to thereby perform gene transfer. Gene transferred cells were selected from retrovirus-added cells by use of 6-ng/ml vincristine. The selected cells were named MCF-7/MDR1.

### (5) Suppression of expression of exogenous P-glycoprotein

Western blotting was performed to investigate expression of exogenous p-glycoprotein in MCF-7/MDR1 cells and effect of estradiol on the expression. A phenol red-free DMEM medium containing fetal bovine serum (7%) which had been treated with activated carbon to remove steroids was used to culture MCF-7 cells and MCF-7/MDR1 cells. Each type of cells were cultured in two dishes. In one of the two dishes, estradiol was added so as to attain a final concentration of 3 nM, and incubation was performed for 4 days. Afterwards, expression level of p-glycoprotein was determined for each cell type using an anti-p-glycoprotein antibody C219 (Fig. 5). Whereas p-glycoprotein was not expressed in MCF-7 cells, MCF-7/MDR1 cells showed strong expression of exogenous p-glycoprotein. In the presence of estradiol, the expression level of exogenous p-glycoprotein in MCF-7/MDR1 cells decreased by about 20% (Fig. 5).

As described hereinabove, a very low level of the steroid hormone, female hormone, or anti-hormone agent reduces expression of BCRP or p-glycoprotein, so that anticancer drug resistance caused by BCRP or p-glycoprotein can be successfully overcome.

## Claims

1. An ABC transporter protein expression inhibitor comprising, as active ingredient(s) thereof, 0.001 to 100 nM of one or more members selected from among steroid hormones, compounds having a female hormone function, analogous compounds thereof and antagonistic inhibitors therefor.

2. The ABC transporter protein expression inhibitor as recited in claim 1, wherein the one or more members selected from among steroid hormones, compounds having a female hormone function, analogous compounds thereof and antagonistic inhibitors therefor are selected from among follicle hormones, corpus luteum hormones, nortestosterones, flavonoids, and analogous compounds thereof.

3. The ABC transporter protein expression inhibitor as recited in claim 1 or 2, wherein ABC transporter expressing cells are cancer cells.

4. The ABC transporter protein expression inhibitor as recited in claim 3, wherein the cancer cells are those which express female hormone receptors.

5. The ABC transporter protein expression inhibitor as recited in claim 3, wherein the cancer cells are breast cancer cell.

6. An anticancer composition containing an ABC transporter protein expression inhibitor as recited in any one of claims 1 to 5 and an anticancer drug.

7. The anticancer composition as recited in claim 6, wherein the anticancer drug is one or more member selected from among camptothecins, anthraquinones, staurosporines, anthracyclines, vinka alkaloids, taxanes, etoposide, mitomycin, gefinitib, and imanitib.

8. The anticancer composition as recited in claim 6 or 7, wherein the cancer is breast cancer.

9. 7-Ethyl-10-hydroxycamptothecin (SN-38) resistant breast cancer cells MCF-7 which express BCRP at high level.

10. 7-Ethyl-10-hydroxycamptothecin (SN-38) resistant breast cancer cells T-47D which express BCRP at high level.

11. Vincristine resistant breast cancer cells MCF-7 which express p-glycoprotein at high level.

12. Vincristine resistant breast cancer cells T-47D which express p-glycoprotein at high level.

13. A method for screening ABC transporter protein expression inhibitors, which comprises using, as an indicator, expression level of BCRP or p-glycoprotein in the 7-ethyl-10-hydroxycamptothecin (SN-38) resistant breast cancer cells or in breast cancer cells exhibiting vincristine resistant breast cancer cells as recited in any of claims 9 to 12.
